# EUROPEAN PATENT SPECIFICATION

(11) **EP 4 370 193 B1**
(45) Date of publication and mention of the grant of the patent: **25.06.2025**
(21) Application number: 22748715.4
(22) Date of filing: 07.07.2022
(51) Int. Cl.: A61N 1/04, A61N 1/05, A61N 1/36, A61N 1/378

(54) **THERAPEUTIC DEVICES FOR STIMULATING NERVES**
THERAPEUTISCHE VORRICHTUNGEN ZUR NERVENSTIMULATION
DISPOSITIFS THÉRAPEUTIQUES DE STIMULATION NERVEUSE

(30) Priority: 12.07.2021 US 202163220828 P; 06.07.2022 US 202217858706
(43) Date of publication of application: 22.05.2024
(73) Proprietor: Verily Life Sciences LLC, Dallas, TX 75019 (US)
(72) Inventor: LEE, Shungneng, San Francisco, California 94080 (US); RAKHYANI, Anil Kumar Ram, San Francisco, California 94080 (US)
(74) Representative: Mewburn Ellis LLP
(86) International application number: PCT/US2022/073504
(87) International publication number: WO 2023/288180

(56) References cited:
- WO-A1-2020/232208
- US-A1- 2020 030 615
- US-A1- 2020 360 692
- US-B2- 10 610 695

## Description

### TECHNICAL FIELD

The present disclosure relates generally to ophthalmic devices, systems, and associated methods for stimulating nerves in and/or around the eye. In particular but not exclusively, the present disclosure relates to devices for placement underneath an eyelid, and out of the field of vision, for stimulating the sclera surface, bulbar conjunctiva, palpebral conjunctiva, and/or eyelid to trigger tear production to treat dry eye.

### BACKGROUND

A large number of people have Dry Eye Disease ("DED"), which includes symptoms of intense pain, stinging eyes, foreign body sensation, light sensitivity, blurriness, increased risk of infection, and possible vision loss. DED is characterized by insufficient tear volume or unbalanced tear composition on the ocular surface of a patient, which is generally caused by insufficient tear production or excessive tear evaporation. Insufficient tear volume results in tear hyperosmolarity, which causes inflammation and nerve damage and can lead to progressive loss of tear production and quality.

Dry-eye symptoms vary based on a variety of factors. For example, dry-eye symptoms vary throughout a day in response to diurnal physiological variations in tear pH, intraocular pressure, corneal sensitivity, visual sensitivity, and melatonin production. For instance, corneal sensitivity is often significantly greater in the evening than compared to the morning. Longer term variations in dry-eye symptoms can be related to use of systemic medications, chronic disease (e.g., diabetes), hormonal changes, and aging. Changes to a patient's environment also contribute to dry-eye symptom variations. For example, dry-eye symptoms can increase due to low humidity of air-conditioned offices, winter heating, computer use, phone use, allergens, and contact lenses.

Some current approaches to treating dry-eye symptoms may not account for the variety of factors that impact the severity and onset of the symptoms, as current treatment for DED is primarily eye drop-based and may provide limited episodic or temporary relief.

Prior art is disclosed by US2020/030615A1, US2020/360692A1, and WO2020/232208A1.

### SUMMARY

The present disclosure describes devices for treating dry eye. According to some aspects, a device is presented that is configured to be positioned underneath an eyelid and worn by a user for treating dry eye. The device includes a first surface configured to face a portion of a bulbar conjunctiva and/or sclera of the eye, and a second surface configured to face the palpebral conjunctiva and/or eyelid. The device further includes a plurality of stimulation electrodes proximal to the first surface, wherein the plurality of stimulation electrodes is configured to stimulate the sclera and/or the bulbar conjunctiva. The electrodes include a plurality of electrode prongs or branches defined by a plurality of slots extending into the electrode regions. The device further includes an antenna and other electronic components to convert electromagnetic energy received from a wireless remote control device into a current and/or voltage to supply power to the plurality of stimulation electrodes. The slots provide a discontinuous or interrupted surface pattern which inhibits the formation of lossy electrical eddy currents in the electrodes to facilitate or promote near-field coupling with the wireless remote control device.

According to the invention as defined by claim 1, a device is provided that is configured to be located underneath an eyelid and worn by a user for treating dry eye. The device includes: a flexible substrate; an antenna disposed on a first side of the substrate and configured to receive electromagnetic energy from a transmitter; one or more electronic components disposed on the first side of the substrate; and a first electrode and a second electrode, the first and second electrodes disposed on a second side of the substrate opposite the first side. In some aspects, each of the first electrode and the second electrode comprises an electrode surface and a plurality of slots in the electrode surface defining a plurality of electrode prongs, where the plurality of slots facilitate near-field coupling between the transmitter and the antenna. In a further aspect, the one or more electronic components are in communication with the antenna, the first electrode, and the first electrode, and the one or more electronic components are configured to provide electrical power to the first electrode and the second electrode.

In some embodiments, the antenna and the one or more electronic components are configured to convert electromagnetic energy into the electrical power. In some embodiments, the one or more electronic components comprises a rectifier. In some embodiments, the one or more electronic components comprises an application-specific integrated circuit (ASIC). In some embodiments, the one or more electronic components comprises one or more discrete surface mount electronic components. In some embodiments, the first electrode comprises a first plurality of electrode prongs, wherein the second electrode comprises a second plurality of electrode prongs, and wherein the first plurality of electrode prongs and the second plurality of electrode prongs extend in opposing directions. In some embodiments, the first plurality of electrode prongs and the second plurality of electrode prongs extend toward a central axis of the flexible substrate. In some embodiments, the first plurality of electrode prongs and the second plurality of electrode prongs extend away from a central axis of the flexible substrate.

In some embodiments, each of the electrode prongs comprises a first width, and wherein each of the slots comprises a second width that is less than the first width. In some embodiments, the first electrode and the second electrode are spaced from each other on the second side of the substrate. In some embodiments, the device further includes an insulating layer disposed on the first side of the substrate, the insulating layer insulating and sealing the antenna. In some embodiments, the antenna comprises a first metallic trace arranged in a spiral. In some embodiments, the antenna defines an antenna region, and wherein the first and second electrodes are juxtaposed on the antenna region. In some embodiments, the first electrode and the second electrode comprise a second metallic trace, and wherein the second metallic trace is insulated from the first metallic trace by the substrate.

According to another embodiment of the present disclosure, a device is provided that is configured to be located underneath an eyelid and worn by a user for treating dry eye. The device includes: a flexible substrate; an antenna trace disposed on a first side of the substrate and configured to receive electromagnetic energy from a remote control device, the antenna trace comprising at least one loop surrounding and defining an antenna region; electronic circuitry electrically coupled to the antenna trace; and a first electrode trace and a second electrode trace electrically coupled to the electronic circuitry, wherein the first and second electrode traces are disposed on a second side of the substrate opposite the first side. In some aspects, each of the first electrode trace and the second electrode trace comprises an electrode surface and a plurality of slots in the electrode surface defining a plurality of electrode branches, wherein the plurality of slots promote near-field coupling between the remote control device and the antenna trace. In a further aspect, the first electrode trace and the second electrode trace are juxtaposed with the antenna region.

In some aspects, the first electrode trace comprises a first plurality of electrode branches extending in parallel. In some aspects, at least a portion of the first electrode trace overlaps with at least a portion of the antenna trace. In some aspects, the device further includes an elastomeric material encapsulating at least the substrate, the electronic circuitry, and the antenna trace. In some aspects, the substrate comprises a bean shape, and wherein device is configured to curl along at least a first axis.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
Figure 1 is a perspective view of a therapeutic device for stimulating nerves in and around a patient's eye, according to aspects of the present disclosure.
Figure 2 is a perspective view of a therapeutic device being worn inside a patient's eyelid and being wirelessly controlled by a remote control device, according to aspects of the present disclosure.
Figure 3 is a top plan view of a therapeutic device for stimulating nerves in and around a patient's eye, according to some aspects of the present disclosure.
Figure 4 is a cross-sectional view of a therapeutic device configured to be worn in the fornix, according to aspects of the present disclosure.
Figure 5 is a cross-sectional view of a therapeutic device configured to be worn in the fornix, according to aspects of the present disclosure.
Figure 6 is a perspective view of a palpebral conjunctiva-facing side of a therapeutic device configured to be worn in the fornix, according to aspects of the present disclosure.
Figure 7 is a diagrammatic view of electronic circuitry of a therapeutic device for stimulating nerves and around a patient's eye, according to aspects of the present disclosure.
Figure 8A is a top plan view of a therapeutic device for stimulating nerves in and around a patient's eye having electrode prongs extending outward, according to some aspects of the present disclosure.
Figure 8B is a top plan view of a therapeutic device for stimulating nerves in and around a patient's eye having electrode prongs extending outward, according to some aspects of the present disclosure.
Figure 8C is a top plan view of a therapeutic device for stimulating nerves in and around a patient's eye having electrode prongs extending inward, according to some aspects of the present disclosure.
Figure 8D is a top plan view of a therapeutic device for stimulating nerves in and around a patient's eye having electrode prongs extending inward, according to some aspects of the present disclosure.
Figure 9A is a top plan view of a first side of a therapeutic device having electrodes disposed on opposing surfaces of a substrate, according to aspects of the present disclosure.
Figure 9B is a top plan view of a second side of the therapeutic device shown in Figure 9A according to aspects of the present disclosure.
Figure 10 is a flow diagram of a method for stimulating nerves using a therapeutic device configured to be worn under a patient's eyelid.
Figure 11 is a top plan view of a therapeutic device for stimulating nerves in and around a patient's eye, according to some aspects of the present disclosure.
Figure 12A is a graphical view of an electrical pulse waveform for use with a nerve stimulation device, according to some aspects of the present disclosure.
Figure 12B is a graphical view of a nerve stimulation waveform for use with a nerve stimulation device, according to some aspects of the present disclosure.

### DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

Disclosed herein are devices for placement underneath the eyelid, and in particular within a patient's fornix, which is a space between the eyelid and the eye. More specifically, the devices described herein may be configured to be positioned between, and directly contacting, the palpebral conjunctiva, and the bulbar conjunctiva. The devices include one surface for facing the eyelid (e.g., palpebral conjunctiva) and another surface for facing the eye (e.g., the sclera and/or the bulbar conjunctiva). In some embodiments, the devices include electrodes configured to stimulate the sclera and/or the bulbar conjunctiva to induce tear production. The devices may be configured to induce electrical currents into the eye tissue or other tissue of the patient at different depths, intensities, and/or frequencies. It may be advantageous for the devices disclosed herein to have relatively small footprints to fit within the confined spaces available within the eyelid, to be flexible and thin to enhance patient comfort, and to generate sufficient voltage and/or current to stimulate the patient's nerve and achieve a desired physiological response.

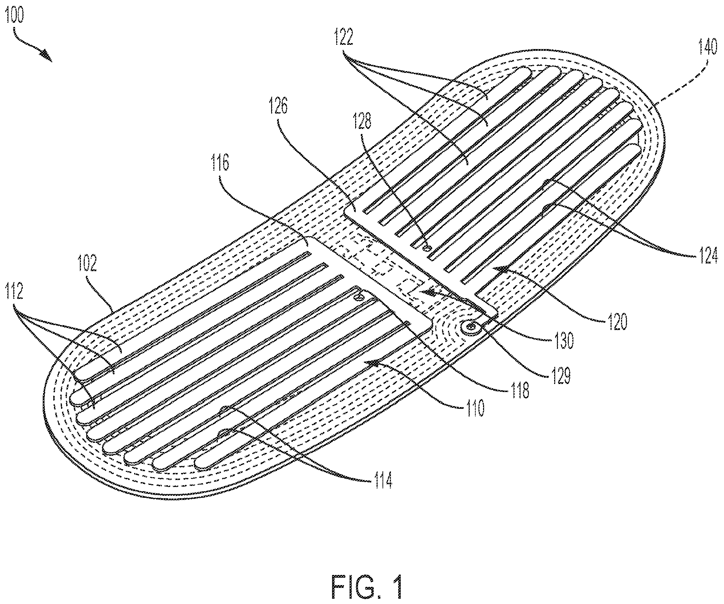

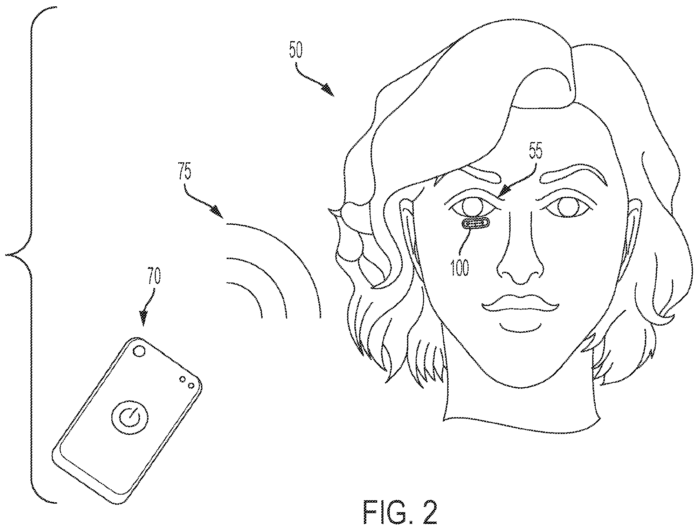

Figure 1 is a perspective view of a therapeutic device 100 configured to be worn underneath a patient's eyelid (e.g., in the fornix) for stimulating nerves in and/or around the patient's eye. The device 100 is flexible along at least one axis and is sized, shaped, and otherwise configured to be worn inside the patient's eyelid, as shown in Figure 2. Referring again to Figure 1, the device 100 includes electrodes 110, 120 configured to stimulate nerves in and around the patient's eye, which may cause or enhance tear production, or other physiological responses to treat dry eye or other ophthalmic conditions.

The device 100 includes a first electrode 110 and a second electrode 120 disposed on a substrate 102. An antenna 140 is disposed on opposing side of the substrate 102 and is configured to receive electromagnetic energy from a wireless device (e.g. remote control 70 shown in Figure 2), and to harness the electromagnetic energy to produce electrical power for the components of the device 100. Electronic components 130 are also attached to the substrate 102 and in electrical communication with the antenna 140 and the electrodes 110, and 120.

The substrate 102 may include a flexible polymer material, such as a liquid crystal polymer (LCP), a polyamide (e.g., KAPTON^{®}), or any other suitable type of substrate. The substrate 102 is configured to bow, bend, or flex along at least one axis, and is configured to electrically isolate the electrical components (electrodes 110, 120, antenna 140) from one another. The substrate 102 may include a single layer of material, or multiple layers of a same material or of different materials. For example, in some embodiments, the substrate 102 includes at least two layers of a polymer material, with the antenna 140 disposed between two of the layers of material. The substrate 102 provides sufficient electrical isolation for the variety of electronic components 130, the electrodes 110, 120, and the antenna 140.

The first electrode 110 comprises a plurality of electrode prongs 112 (which may also be referred to as electrode fingers or branches) separated or defined by a plurality of slots 114. The slots 114 extend from an outer region or boundary of the electrode 110 towards an inner or central portion of the electrode 110, where the prongs 112 are electrically connected by an electrode spine 116. Similarly, the second electrode 120 comprises a plurality of electrode prongs 122, where the electrode prongs 122 are separated or defined by a plurality of slots 124. The slots 124 extend from an outer region or boundary of the electrode 120 towards an inner or central portion of the electrode 120, where the prongs 122 are electrically connected by an electrode spine 126. The electrodes 110, 120 are each in electrical communication with the electronic components 130 and the antenna 140, but may be otherwise electrically isolated from one another.

In some aspects, the first electrode 110 and the second electrode 120 may be described as including electrode surfaces defined by the outline or footprint of each electrode 110, 120. In this regard, the slots 114, 124 may be referred to as extending into each respective electrode surface. In the illustrated embodiment, the electrodes 110, 120 are oriented such that the electrode prongs 112, 122 extend outward in opposing directions away from a central axis of the substrate 102. However, as will be further explained below, the electrodes 110, 120 may be oriented such that the electrode prongs 112, 122 extend inward in opposing directions toward the central axis of the substrate 102. In other embodiments, the electrode prongs 112, 122 may extend in parallel toward a top side of the device 100, or a bottom side of the device. In other embodiments, the electrode prongs 112, 122 extend in a same direction, such as to the right or to the left.

The electrodes 110, 120 may be sized and shaped to occupy a significant portion of the footprint of the loop antenna 140. This regard, it may be advantageous for each electrode 110, 120 occupy a relatively large portion of the total surface area of the device 100. In this regard, stimulation of the nerves may be improved by greater contact areas of the electrodes 110, 120 and the patient's tissue/nerves. However, a large conductive surface overlapping with the antenna 140 may interfere with the antenna's 140 ability to harness electromagnetic (EM) energy wirelessly to provide to the electrodes 110, 120 and other electronic components 130 of the device 100. For example, electrical eddy currents may result from electromagnetic energy fields passing through the antenna 140. However, the slots 114, 124 reduce, limit, or eliminate the formation of eddy currents such that the antenna 140 can more efficiently harness electrical power for the components of the device 100. The slots 114, 124 may be wider or narrower than what is shown in the embodiment of Figure 1. However, it may be advantageous for the slots 114, 124 to be very narrow (e.g., 25 µm - 150 µm) to maintain a proportionally high surface area for each electrode 110, 120. Accordingly, the slots and prongs configuration of the electrodes 110, 120 promote near-field coupling between the device 100 and a wireless remote control device (e.g., 70, Figure 2).

The slots 114, 124 may extend into the electrode regions of the electrodes 110, 120 by a greater or lesser amount than what is shown in Figure 1. Further, although the slots 114, 124 are shown as straight and parallel to one another, it will be understood that the slots 114, 124 may have arcuate profiles, zigzag profiles, curved profiles, non-parallel profiles, or any other suitable shape. Further, the electrodes 110, 120 may define shapes or footprints other than what is shown. For example, each electrode may define a circular, or roughly circular electrode region, with the slots 114, 124 extending into the respective electrode regions. In other embodiments, the electrodes 110, 120 may define rectangular regions, elliptical regions, bean-shaped regions, triangular regions, hexagonal regions, or any other suitable shape. In some embodiments, the electrodes 110, 120 overlap with the antenna 140 by a greater or lesser extent than what is shown in Figure 1. For example, one or both of the electrodes 110, 120 may overlap an entirety of the loop antenna 140 in at least one direction (e.g., width). In other embodiments, one or both of the electrodes 110, 120 may be sized and shaped to fit entirely within an innermost loop of the antenna 140, such that no portion of the electrodes 110, 120 overlaps with any portion of the antenna 140. In other embodiments, one or both of the electrodes 110, 120, may extend beyond a footprint of the antenna 140 in at least one direction.

The electrodes 110, 120 may comprise metallic traces, films, or foils disposed on the substrate 102. For example, the electrodes 110, 120 may be disposed on the substrate 102 by chemical vapor deposition, sputtering, laser welding, mounting, adhesion, manual fabrication, or any other suitable process. The electrodes 110, 120 may include a biocompatible conductive material, such as gold, platinum, iridium, alloys that include gold, platinum, and/or iridium, or any other suitable material. Similarly, the antenna 140 may comprise one or more traces of a conductive material, such as gold, platinum, iridium, or alloys thereof. For example, in some embodiments the antenna 140 and the electrodes 110, 120 comprise a same type of material in other embodiments, the electrodes 110, 120 comprise a different material in the antenna 140.

In the illustrated embodiment, the antenna 140 includes a spiral of metallic traces having four loops. The four loops may be concentric and non-overlapping such that the antenna 140 can be deposited on the substrate 102 in a single manufacturing step. However, it will be understood that in other embodiments, the loop antenna 140 may differ from what is shown in one or more aspects. For example, the loop antenna 140 may include fewer or more loops than what is shown in Figure 1. In some embodiments, the loop antenna 140 includes a single loop of metallic material. In some embodiments, the loop antenna 140 may include multiple electrically connected traces that are not disposed in a spiral shape. For example, the antenna 140 may include distinct concentric loop traces. The antenna 140 may occupy a greater or lesser amount of the available surface area on the device 100 than what is shown in Figure 1. Further, although the antenna 140 defines a kidney or bean shape, it will be understood that the antenna 140 may define other shapes, such as elliptical, circular, rectangular, triangular, or any other suitable shape. The antenna 140 is disposed on an opposite side of the substrate 102 than the electrodes 110, 120. Accordingly, although the electrodes 110, 120 overlap with the antenna 140, the electrodes 110, 120 are isolated from the antenna 140 by the substrate 102.

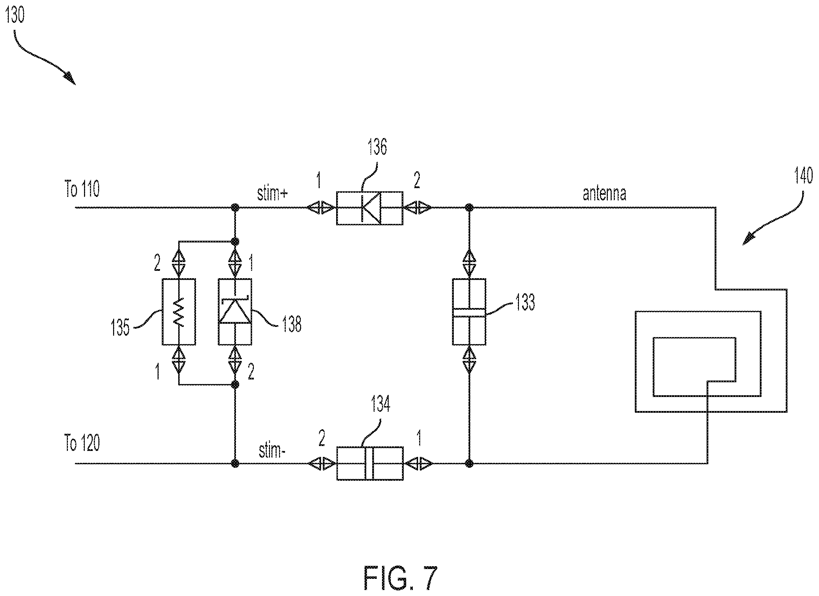

The device 100 includes a plurality of vias 118, 128, 129, which provide points of electrical connection between the electrodes 110, 120, and the electronic components 130 and/or the antenna 140, which are disposed on the opposite side of the substrate 102 The vias 118, 128, 129 may include a hole or aperture extending through the substrate 102, where the walls or interior surfaces defining the aperture are covered by a conductive material, such as hold, platinum, iridium, alloys thereof, or any other suitable conductive material. The electrical connections between the electrodes 110, 120, the electronic components 130, and the antenna 140 will be described further below with respect to Figure 7.

The electronic components 130 may include or provide an electrical rectifier to modulate the electromagnetic energy provided by the antenna 140 into direct current. Further, the electronic components 130 may include electronic control components to selectively activate one or both of the electrodes 110, 120. The electronic components 130 may be connected to each other, to the electrodes 110, 120, and/or to the antenna 140 by one or more conductive traces, filars, or other conductors coupled to or disposed on the substrate 102. In some embodiments, the electronic components 130 may be contained or packaged into a single chip, such as an application-specific integrated circuit (ASIC). In some embodiments, the electronic components 130 include one or more discrete surface mount components, such as capacitors, resistors, diodes, inductors, transistors, and/or any other suitable discrete surface mount component. In some embodiments, the electronic components 130 include one or more ASICs in addition to one or more discrete surface mount components. The electronic components 130 may further include electronics that improve or increase the safety of the device. The embodiment shown in Figure 1 is configured for battery-less operation in which the device 100 is powered solely by a wireless device. Battery-less operation may allow for a smaller and more comfortable form factor. In other embodiments, the device 100 may include a battery to allow for occasional nerve stimulation according to a configured schedule stored in a memory of an ASIC.

The device 100, which is configured to be worn under the patient's eyelid, may be configured to stimulate the bulbar conjunctival surface to efferently activate lacrimal gland secretion. Because the nerve density on the bulbar conjunctiva is relatively sparse, it may be advantageous to use electrodes having a relatively large surface area. The device 100 allows for larger electrodes to be used without degrading the radiofrequency (RF) coupling and power transfer with the antenna 140 for operation of the device 100. As described further below, the device 100 may also include a magnetic waveguide backing material to further improve RF power coupling efficiency. The improved efficiency may allow for a handheld remote control device to have a longer lifetime between recharges. Further, lower RF power may be used to operate the device to limit the amount of electromagnetic radiation directed towards the patient. The larger electrodes sizes allow for larger stimulation charge for a given charge density limit, and may cover more nerves (e.g., ciliary nerves) for improved treatment. Moreover, the slotted electrodes 110, 120 may allow for the creation of spatial field patterns that can increase the efficacy of nerve stimulation by using spatial field gradients near nerves or cells.

Figure 2 is a perspective view of a patient 50 wearing a nerve stimulation device 100 inside the bottom eyelid below the eye 55. The device 100 is being controlled by a remote control device 70, which emits electromagnetic energy 75 toward the device 100. In some aspects, the remote control device 70 may be referred to as a transmitter or a controller. The remote control device 70 is configured to emit the electromagnetic energy 75 according to a predefined protocol. The protocol used to emit the electromagnetic energy 75 may determine which of the electrodes 110, 120 of the device 100 is activated, for what amount of time, and/or at what intensity. The remote control device 70 may resemble a wireless car key fob or an optical thermometer, in some embodiments. The remote control device 70 may be powered by an internal battery, and may be compact and portable such that the patient 50 can carry the remote control device 70 with her wherever she goes. Accordingly, the patient 50 may use the remote control device 70 to activate the electrodes 110, 120 of the device 100 at the onset of dry eye symptoms, for example. In some aspects, the patient 50 may use the remote control device 70to activate the device 100 according to a predefined or prescribed schedule.

The remote control device 70 may be configured to be worn as a necklace in some aspects. In some embodiments, the wireless remote control device 70 may include a smart phone device configured with NFC capabilities. In some aspects, the remote control device 70 may record usage data, and/or ensure patient safety. In some aspects, the remote control device 70 may be configured to run an app that helps with disease management and compliance.

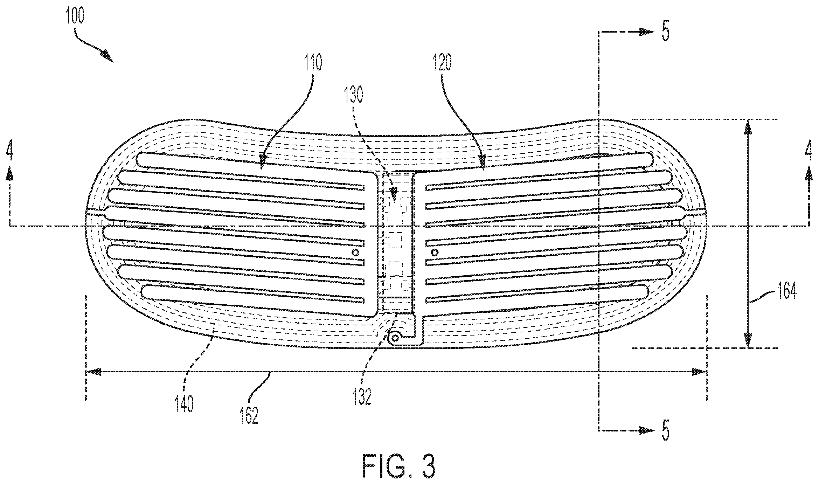

Figure 3 shows a top plan view of the device 100. The device 100 has a bean shape or kidney shape. The shape of the device 100 may correspond to the available space within the patient's fornix, which is the space between the eyelid and the surface of the eye. Further, the shape of the device 100 may be selected accounting for curvature of the device 100 in at least one axis while being worn by a patient. In some aspects, the bean shape may allow the device 100 to conform to the volume available in the fornix, and to conform to the curvature of the cornea to facilitate contact of the electrodes with the bulbar conjunctiva, sclera, and/or the limbus. In some aspects, the device 100 may be used to stimulate the limbus, if the electrode separation is sufficient and occupies more of the volume available in the fornix.

A large portion of the footprint of the device 100 is occupied by the electrodes 110, 120. A plurality of electronic components 130 is disposed in an electronics region 132 between the electrodes 110, 120. In other embodiments, the electronics region 132 may be positioned below, above, or to the right or to the left of the electrodes 110, 120.

The device 100 includes a width 162 and a height 164. The width 162 height 164 and shape of the device 100 may define or determine the size, area, or footprint of the device. In this regard, the width 162, height 164, shape, and overall footprint of the device 100 is suitable for positioning under the patient's eyelid and within the fornix. For example, the width 162 of the device 100 may range between 5mm and 25 mm, and the height 164 of the device 100 may range between 2 mm and 15 mm. In this regard, the area or footprint of the device 100 may range between 10mm² and 400 mm². Further, the electrodes 110, 120 may occupy, in the aggregate, a substantial portion of the area or footprint of the device 100. For example, the electrodes 110, 120, taken together, may occupy more than 50 percent of the total area or footprint of the device. In the illustrated embodiment, the overall size or footprint of the device 100 substantially corresponds to the size of footprint of the antenna trace 140. However, in some embodiments, the device has a footprint which is substantially larger than the footprint of the antenna 140. For example, in some embodiments, the regions occupied by the electrodes 110, 120 extend past or beyond the antenna region defined by the antenna trace 140 in one or more directions. It will be understood that the dimensions and ranges described herein are exemplary only and are not limiting. For example, the device 100 may include a width 162, height 164, or other dimension greater or smaller than what is explicitly stated herein.

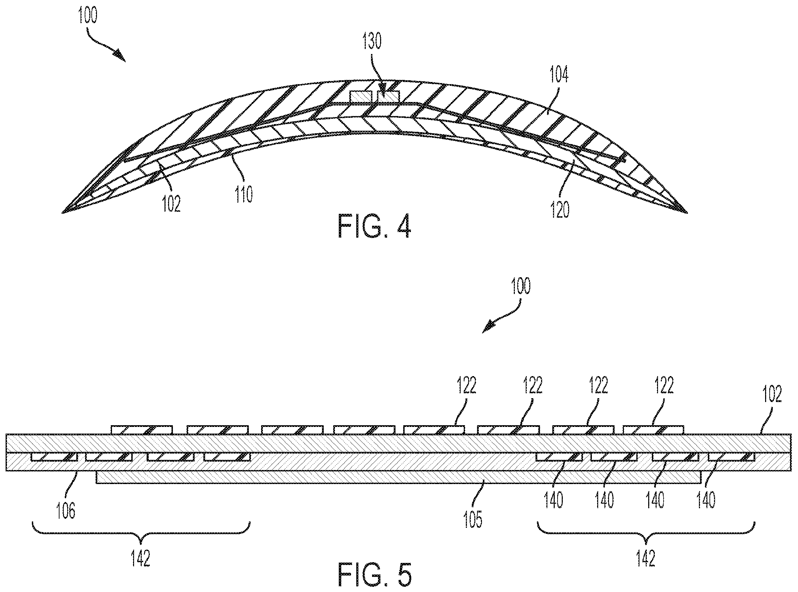

Figures 4 and 5 are cross-sectional views of the device 100 shown in Figure 3, taken along lines 4-4 and 5-5, respectively. Referring to Figure 4, the device 100 is shown in a curved state after being inserted into the patient's fornix. The electronic components 130 are disposed on a palpebral conjunctiva-facing side, which may also be referred to as a posterior side, a back side, or a rear side of the device 100 away from the patient's eyeball, and the electrodes 110, 120 are on an opposite bulbar conjunctiva-facing side of the device 100 such that the electrodes 110, 120 are in electrical communication with the eye tissue. The device 100 also includes a flexible coating or encapsulant 104, with the substrate 102, electronic components 130, and electrodes 110, 120 embedded within and surrounded by the encapsulant 104. The encapsulant 104 may comprise a biocompatible elastomeric material, such as a silicone elastomer, hydrogel, silicone hydrogel, or any other suitable biocompatible material. In some embodiments, the encapsulant 104 includes an LCP, polyimide, or any other suitable polymeric material. In some embodiments, the electrodes 110, 120 are not covered or encapsulated by the encapsulant 104 such that the electrodes can make direct physical contact with the patient's eye. In other embodiments, the electrodes 110, 120 are covered by or encapsulated in the encapsulant 104, but at least the portion of the encapsulant 104 disposed over the electrodes is conductive such that the electrodes 110, 120 can stimulate the nerves through the encapsulant 104.

Referring to Figure 5, a cross-sectional view of the device 100 is shown without the encapsulant 104. The electrode prongs 122 of the second electrode 120 are shown disposed on a first side of the substrate 102, and the antenna trace 140 is disposed on an opposite second side of the substrate 102, such that the substrate 102 electrically insulates the electrode prongs 122 from the antenna trace 140. Further, the outer prongs 122 overlap the antenna trace region 142, which corresponds to a total width of the antenna trace 140 on either side of the loop. In the embodiment of Figure 5, a coating or insulating layer 106 is disposed over the antenna trace 140 and the second side of the substrate 102. In some embodiments, the coating layer 106 is the encapsulant 104. In other embodiments, the coating layer 106 comprises a different layer and/or material. For example, the coating layer 106 may include an LCP or a polyimide layer bonded to the substrate 102. In this regard, the coating layer 106 may include the same material as the substrate 102 or a different material.

Further, in the embodiment shown in Figure 5, a magnetic waveguide backing material 105 is coupled to a rear side or backside of the insulating layer 106. The magnetic waveguide backing material 105 can guide the incident magnetic fields generated by the remote control device (70, Figure 2) away from the large electrodes 110, 120, resulting in lower power losses in the electrodes 110, 120 this may result in improving the efficiency of wireless power linking between the remote control device and the antenna 140. Increase in efficiency due to the magnetic backing material 105 may depend on the amount and location of the magnetic material used. Typically, the properties of the ferrite material may be < 150 for real permeability and < 5 for imaginary permeability at the frequency of interest. It will also be understood that the magnetic waveguide backing material 105 may be coupled to the substrate 102 and/or the insulating layer 106 in a configuration different from what is shown. For example, in some embodiments, the magnetic waveguide backing material 105 may be disposed on the opposite second side of the substrate 102, opposite the electrode prongs 122, with the antenna 140 and the insulating layer 106 disposed over the magnetic waveguide backing material 105. In other embodiments, the magnetic waveguide backing material 105 is disposed directly over the antenna 140, and between the substrate 102 and the insulating layer 106. In other embodiments, the device 100 does not include the magnetic backing material 105. In some embodiments, the magnetic waveguide backing material 105 includes a flexible ferrite sheet. The flexible ferrite sheet may include a ferrous material to facilitate magnetic field coupling. One example of a flexible ferrite sheet is a MCP-DS-MHLL Sheet manufactured by Laird Technologies Inc.

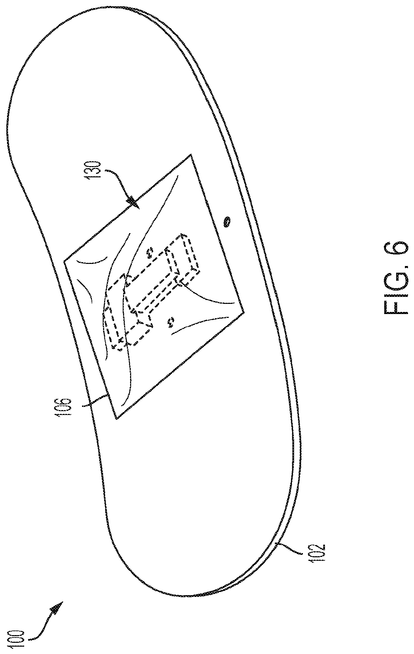

Figure 6 is a perspective view of a palpebral conjunctiva-facing side of the device 100, which may also be referred to as a rear side, a posterior side, or a back side. In the embodiment shown in Figure 6, the palpebral conjunctiva-facing side of the device 100 includes the insulating layer 106 positioned over the substrate 102 and the electronic components 130. The insulating layer 106 may be attached, adhered, deposited, or otherwise coupled to the substrate 102 such that the insulating layer 106 insulates the electronic components 130. In some embodiments, the insulating layer 106 also insulates, covers, and/or seals the antenna trace 140 (Figure 5). In other embodiments, the antenna trace 140 is insulated by a different insulating layer or substrate layer, and the insulating layer 106 is disposed over the different insulating layer or substrate layer.

The electronic components 130 are mounted to or deposited on the palpebral conjunctiva-facing side of the device 100. In some aspects, placing the bulging or protruding electronic components 130 on the palpebral conjunctiva-facing side of the device 100 may improve patient comfort, as the protruding electronic components 130 will be positioned away from the patient's eye tissue when the device 100 is inserted into the patient's fornix. The insulating layer 106 may be heat shrunk, stamped, or otherwise deformed to accommodate the electronic components 130 and provide for a more comfortable, or smoother surface. In other embodiments, the electronic components 130 are mounted to or deposited on the bulbar conjunctiva-facing side, or front side, of the device 100.

Figure 7 is a diagrammatic view of electronic components 130 of the therapeutic device, according to an embodiment of the present disclosure. The electronic components 130 include capacitors 133, 134, diodes 136, 138, and a resistor 135 in electrical communication with the antenna 140 and the electrodes 110, 120. In some aspects, the antenna 140 shown in Figure 7 may be referred to as a loop inductor. The electronic components 130 may provide one or more electrical rectifiers configured to convert pulsed RF waves provided by the antenna 140 to pulsed electrical energy to power the electrodes 110, 120. For example, the electronic components 130 may be configured to provide current pulses or voltage pulses to one or both of the electrodes 110, 120. In some embodiments, the electronic components 130 may be configured to provide biphasic pulses. In other embodiments, the electronic components 130 are configured to provide monophasic pulses.

It will be understood that the embodiment shown in Figure 7 is for illustrative purposes only, and that the electronic components 130 may vary from what is shown. For example, in some aspects, the electronic components 130 are included in one or more application specific integrated circuits (ASICs) configured to rectify the electrical energy provided by the antenna 140. In other embodiments, the electronic components 130 may include fewer or more components than what is shown in Figure 7.

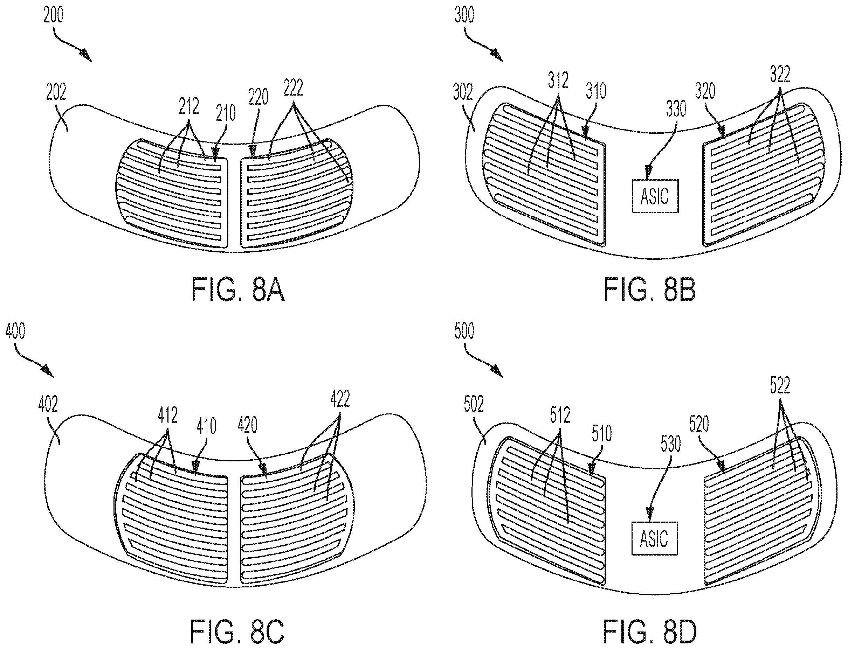

Figures 8A-8D are top plan views of nerve stimulating devices 200, 300, 400, 500, according to various embodiments of the present disclosure. In particular, the embodiments shown in Figures 8A-8D illustrate nerve stimulating devices according to various electrode prong and spacing configurations. In this regard, the electrodes (e.g., 210, 220) may be positioned close together, or spaced apart. Further, the electrode prongs (212, 222) may extend outward away from a central axis of the device, or may extend inward toward the central axis of the device.

Referring to Figure 8A, the device 200 includes electrodes 210, 220 mounted on a substrate 202. The electrodes 210, 220 comprise respective sets of prongs 212, 222 extending away from a central axis of the device 200. In other words, the electrodes 210, 220 include slots extending into respective electrode surfaces from the outer boundaries of the electrode surfaces. Further, the electrodes 210, 220 may be described as being relatively close to one another with respect to the central axis of the device 200. In some embodiments, the spacing between the electrodes 210, 220 may be between 10% and 200% of the width of each electrode 210, 220.

Referring to Figure 8B, the device 300 includes electrodes 310, 320 mounted on a substrate 302. The electrodes 310, 320 comprise respective sets of prongs 312, 322 extending away from a central axis of the device 300, similar to the device shown in Figure 8A. In other words, the electrodes 310, 320 include slots extending into respective electrode surfaces from the outer boundaries of the electrode surfaces. Further, the electrodes 310, 320 may be described as being relatively spaced apart from one another with respect to the central axis of the device 300. In some embodiments, the spacing between the electrodes 310, 320 may be between 10% and 200% of the width of each electrode 310, 320.

Referring to Figure 8C, the device 400 includes electrodes 410, 420 mounted on a substrate 402. The electrodes 410, 420 comprise respective sets of prongs 412, 422 extending inward toward a central axis of the device 400. In other words, the electrodes 410, 420 include slots extending into respective electrode surfaces from the inner boundaries of the electrode surfaces. Further, the electrodes 410, 420 may be described as being relatively close to one another with respect to the central axis of the device 400, similar to the device 200 shown in Figure 8A.

Referring to Figure 8D, the device 500 includes electrodes 510, 520 mounted on a substrate 502. The electrodes 510, 520 comprise respective sets of prongs 512, 522 extending inward toward a central axis of the device 500, similar to the device 400 shown in Figure 8C. In other words, the electrodes 510, 520 include slots extending into respective electrode surfaces from the inner boundaries of the electrode surfaces. Further, the electrodes 510, 520 may be described as being relatively spaced apart from one another with respect to the central axis of the device 500, similar to the device 300 shown in Figure 8B.

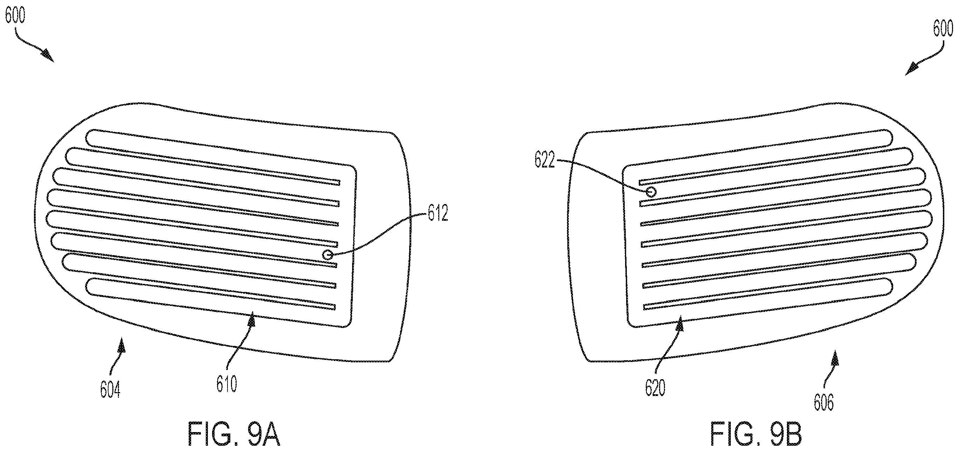

Figures 9A and 9B illustrate a nerve stimulation device 600 according to another embodiment of the present disclosure. In the illustrated embodiment, the device 600 includes a first electrode 610 mounted on a first side 604 of the device 600, and a second electrode 620 mounted on an opposite second side 606 of the device 600. In some aspects, the first side 604 may be a palpebral conjunctiva-facing side, and the second side 606 may be a bulbar conjunctiva-facing side. The electrodes 610, 620 may be mounted to one or more substrates comprising one or more layers of polymeric material. In particular, the first electrode 610 may be mounted on a first substrate layer, with an antenna attached to, coupled to, or mounted on an opposite second side of the first substrate layer. Further, a second substrate layer may be placed over an opposite side of the antenna, with the second electrode 620 mounted to the opposite second side of the second substrate layer. Accordingly, the antenna may be positioned between two substrate layers, with the electrode 610, 620 mounted to opposite outward-facing sides of the respective substrate layers. Further, electrical circuitry may be positioned between the electrode layers. The device includes a first electrical via 612 on the first side 604, and a second electrical via 614 on the second side 606. The electrical vias 612, 622 may extend through the respective substrate layers to electrically connect the electrodes 610, 620 to the electronic components and/or to the antenna trace between the respective substrate layers.

In the embodiment shown in Figures 9A and 9B, the path of the current created by the electrodes 610, 620 disposed on opposite facing sides of the device 600 may be more shallow or superficial compared to other electrode configurations (e.g., electrodes 110, 120). Accordingly, the device 600 may be more suitable or appropriate for stimulating specific types of nerves which are more superficial. For example, the device 600 may be used to stimulate nerves in the palpebral conjunctiva, namely the meibomian glands, or along with adjusting the stimulation waveforms, both the meibomian glands in the palpebral conjunctiva and the afferent nerves governing reflex tearing in the bulbar conjunctiva and sclera. Although the device is shown as having a partially curved outer profile, it will be understood that the device 600 may include other shapes, footprints, or profiles than what is shown. For example, the device 600 may include a rectangular profile, a circular profile, and elliptical profile, a triangular profile, a bean-shaped profile, or any other suitable profile. Similarly, the electrodes 610, 620 may include profiles other than what is shown, and may or may not correspond to or match the overall profile of the device 600. For example, the electrodes 610, 620 may include circular profiles, elliptical profiles, rectangular profiles, triangular profiles, bean-shaped profiles, or any other suitable profile.

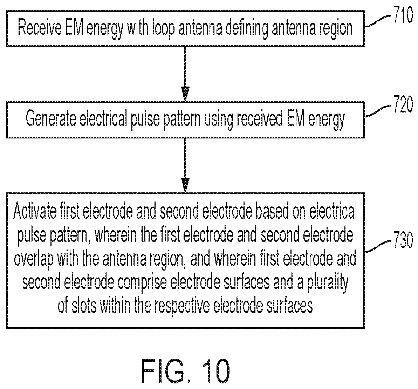

Figure 10 is a flow diagram illustrating a method 700 for stimulating nerves using a nerve stimulation device. In this regard, the method 700 may be performed using any of the devices 100, 200, 300, 400, 500, 600, 800 described herein. In particular, the method 700 may be performed using a nerve stimulation device that includes an antenna configured to receive electromagnetic energy, one or more electrodes configured to create an electrical current in the patient's tissue, and electronic circuitry configured to convert the electrical energy received by the antenna into electrical power to be used by the one or more electrodes. Further, the method 700 may be performed using a wireless remote control device, such as the remote control device 70 shown in Figure 2.

At block 710, a nerve stimulation device receives electromagnetic (EM) energy with a loop antenna defining an antenna region. The EM energy may be provided by a remote control device, which emits a varying EM field according to a predefined protocol or waveform. As the varying EM field passes through the antenna region, the varying EM field induces a current in the antenna, which is provided to the electronics of the device.

At block 720, the device, specifically the electronic circuitry of the device, generates an electrical pulse pattern based on the received EM energy. In this regard, the electrical pulse pattern may correspond to the predefined protocol or waveform emitted by the remote control device. The electrical pulse pattern may include a variety of characteristics, such as a pulse, a pulse intensity, a pulse frequency, electrode polarity, or any other suitable characteristic. In some aspects, the electrical pulse pattern may be created using a rectifier of the electronic circuitry to convert AC electrical current provided by the antenna into DC electrical current.

At block 730, the device activates a first electrode and a second electrode based on the electrical pulse pattern the first electrode and second electrode overlap with the antenna region defined by the antenna. The first electrode and second electrode comprise electrode surfaces and a plurality of slots within the respective electrode surfaces. The slots extending with in the electrode surfaces may promote near-filed coupling between the antenna of the nerve stimulation device and the remote control device. For example, the slots, which separate the electrodes into a plurality of electrode prongs or fingers, may limit or inhibit the creation of lossy electrical eddy currents which can compromise efficiency and power transfer. Accordingly, by including the slots in the electrodes, the size or footprint of the electrode surfaces can be increased to substantially or entirely overlap with the antenna region, with less interference with the antenna's harnessing of the EM energy.

In some aspects, the wireless remote control device may be configured with smart stimulation features. For example, in the method 700, the wireless remote control device may include a smart phone, or may provide for wireless connectivity with the smart phone (e.g., Bluetooth) using a smartphone app. The remote control device may include a variety of stimulation waveforms for magnetic pulsing and algorithms. A handheld wand may include various treatment tracking features, such as an accelerometer to track the remote control device's treatment motion, and/or a wireless connection with a cellphone to give better treatment advice (figure out where "blindspots" are in treatment). The wireless remote control device may track treatment time(s) and duration, and send reminders.

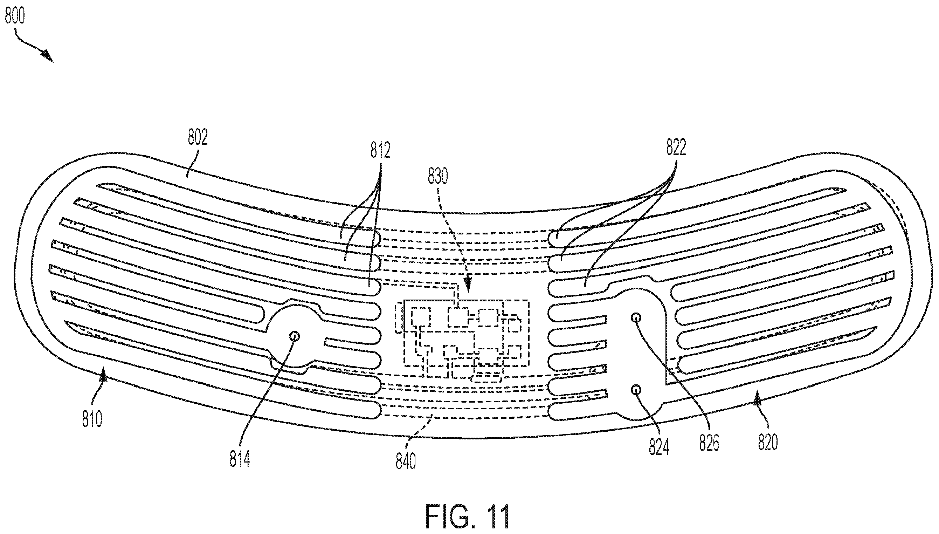

Figure 11 is a top plan view of a therapeutic device 800 configured to be worn in a patient's fornix. Similar to the devices described above, the device 800 includes a first electrode 810 and a second electrode 820 disposed on or mounted to a substrate 802. Each electrode 810, 820 includes a plurality of electrode prongs or branches 812, 822 separated from each other by a plurality of slots. The electrode prongs 812, 822 extend inward in opposing directions toward electronic components 830 mounted in a central region of the substrate 802. The device 800 further includes an antenna trace 840 disposed on the substrate 802. The antenna trace 840 and the electronic components 830 are disposed on a palpebral conjunctiva side, or posterior side, of the substrate 802, while the electrodes 810, 820 are disposed on a bulbar conjunctiva side, or interior side, of the substrate 802. The device 800 has a bean shape or kidney shape. The shape of the device 800 may be selected accounting for curvature of the device 800 in at least one axis while being worn by a patient. Further, the electrodes 810, 820 are curved upward at the outer regions of the electrodes 810, 820. In some aspects, the wider dimension of the device 800, the spaced configuration of the electrodes 810, 820, and the curvature of the electrodes 810, 820 may improve the current density distribution between electrodes to recruit more nerves at a uniform current density. Because the wider dimensions also allow for improved current density distribution vs. volume of tissue, increased neuronal recruitment, and greater curvature of the device 800, the illustrated configuration also penetrates the eye tissue further due to the curved structure of the electrodes 810, 820 around the eye. Furthermore, the upward curvature of the electrodes may provide for stimulation of nerves closer to the limbus, where there is higher nerve density, which allows for more efficient stimulation.

The device 800 includes a first electrical via 814 on the first electrode 810, and a second electrical via 824 and third electrical via 826 on the second electrode 820. The electrical vias 814, 824, 826 may include an aperture or opening in in the electrodes 810, 820, and through the substrate 802 to the electronic components 830 on the other side of the substrate 802. The vias 814, 824,826 may include a metallic layer or coating (e.g., gold, copper, platinum, iridium, etc.) extending through the aperture or opening, which is connected to an electrical trace leading to a corresponding component of the electronic components 830. The vias 814, 824, 826 include conductive ring areas that are wider than the prongs 812, 822. The vias 814, 824, 826 are positioned within the pronged or branched electrodes 810, 820, such that the vias 814, 824, 826 interrupt the pattern of parallel or co-extending electrode prongs 812, 822. In this regard, the electrodes 810, 820, include a break in at least one prong 812, 822 such that each via 814, 824, 826 is directly connected to a single prong 812, 822 on an outer side of each via, and two or more prongs 812, 822 on an inner side of each via (i.e., closer to the electronic components 830). The interrupted configuration of the prongs 812, 822 and vias 814, 824, 826 shown in Figure 11 may promote or facilitate near-field coupling between the antenna trace 840 and a remote control device by inhibiting the formation of electrical eddy currents in the conductive layer that includes the electrodes 810, 820 and the vias 814, 824, 826. The location of the vias 814, 824, 826 within the electrodes 810, 820 may allow for sufficient space from the electronic components 830, which may be advantageous for manufacturing purposes.

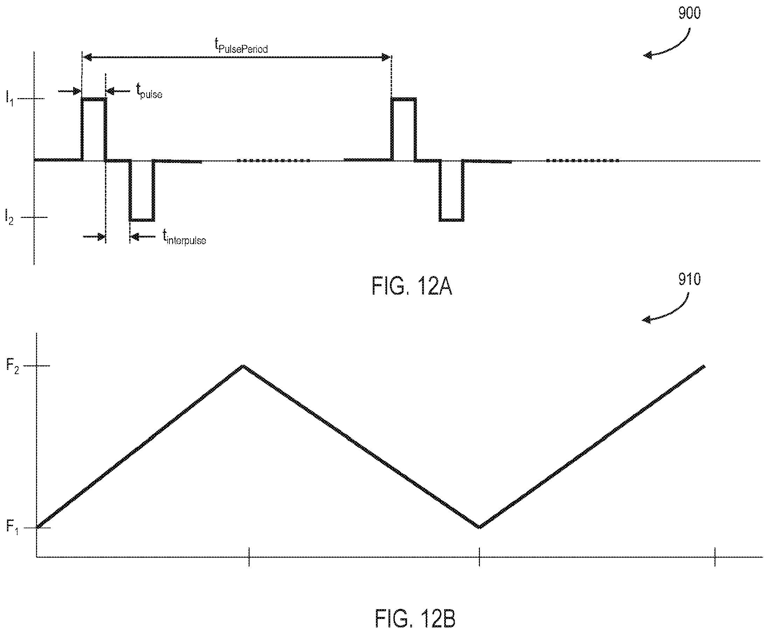

Figures 12A and 12B are diagrammatic views of electrical pulse waveforms 900, 910 for use in treating ophthalmic conditions, such as DED. In this regard, the electrodes of a nerve stimulation device can be pulsed (e.g., using wireless remote control device 70, Figure 2) according to a predefined pattern or waveform to stimulate nerves in and around the patient's eye to induce tear production. Referring to Figure 12A, a nerve stimulation device may be configured to provide a biphasic pulse having a first phase with a positive current I₁, and a second phase with a negative current I₂. The waveform 900 may be described or defined by a pulse width tₚᵤₗₛₑ, an inter-pulse width tᵢₙₜₑᵣₚᵤₗₛₑ, and a pulse period t_{PulsePeriod}, which is the period of time between pulses of the same phase. In some aspects, the pulse period t_{PulsePeriod} is inversely related to the frequency of the pulse waveform (e.g., inter-pulse frequency = 1/t_{PulsePeriod}). In some embodiments, I₁ may range between 0 to 3.3 mA, and I₂ may range between 0 to -3.3 mA. The current values depend on a variety of other variables, including but not limited to electrode area, tₚᵤₗₛₑ, electrode material, etc.. Further, the tₚᵤₗₛₑ may range from approximately 10 µs to 1ms. In one example, tₚᵤₗₛₑ is 100 µs. In some embodiments, tᵢₙₜₑᵣₚᵤₗₛₑ may range from about 10 µs to 1ms. In one example, tᵢₙₜₑᵣₚᵤₗₛₑ is 10 µs. It will be understood that these values are merely exemplary and that other values for I₁, I₂, t_{PulsePeriod}, tₚᵤₗₛₑ, and/or tᵢₙₜₑᵣₚᵤₗₛₑ may be used within the scope of the present disclosure.

The waveform 900 shown in Figure 12A may be repeated for a number of pulses, and for a period of time. During the period of time, the pulse period t_{PulsePeriod} may vary. For example, the pulse period t_{PulsePeriod} may increase or decrease from pulse to pulse, or a same t_{PulsePeriod} may be used for multiple pulses before changing incrementally over the period of time. In the waveform 910 shown in Figure 12B, for example, the pulse period t_{PulsePeriod} changes such that the frequency of the pulses, which is based on the inverse of the pulse period (1/t_{PulsePeriod}), increases and decreases in a linear fashion between F₁ and F₂.

The frequencies that are effective for simulating the nerves and inducing tear production may vary from patient to patient. Determining the most effective pulse frequency (t_{PulsePeriod}) for each individual patient may be impractical in some aspects. Accordingly, the waveforms 900, 910 shown in Figures 12A and 12B provide a widely-applicable pulsing pattern, which sweeps through a plurality of pulse frequencies between F₁ and F₂ in a cyclical fashion. The range of frequencies from F₁ to F₂ may include nerve pulsing frequencies that are at least partially effective for a large portion of the public, a majority of the public, or even the entirety of the public. The waveform 910 may be performed over a time window, in which each frequency between F₁ and F₂ is pulsed at least two times, at least three times, or more times. In other aspects, the waveform may pulse each frequency between F₁ and F₂ a single time.

In one example, F1 may be about 20 Hz and F2 may be about 640 Hz. Accordingly, referring to the waveform shown in Figure 12B, the electrodes may be pulsed at 20 Hz initially, and then increase linearly from 20 Hz to 640 Hz, decrease linearly to 20 Hz, and then increase linearly again to 640 Hz. In some aspects, the entire time window used for the pulsing waveform 910 shown in Figure 12B may be about 1.5 s. However, it will be understood that these values are merely exemplary, and that any suitable values may be used for F₁ and F₂, including frequencies larger or smaller than those explicitly mentioned. Further, in other embodiments, the nerve stimulation device may be configured to increase and/or decrease the pulse frequencies non-linearly.

The various parameters of the waveforms 900, 910 described with respect to Figures 12A and 12B may be determined or controlled by a wireless remote control device or transmitter, in some embodiments. For example, referring to Figure 2, the remote control device 70 may include a voltage source (e.g., a battery) and an inductor circuit comprising one or more resistors and/or capacitors and a loop inductor, a processor, and a memory component having program code or instructions stored thereon. In some aspects, the remote control device 70 may include a power source, which may include a battery, and the power source may be configured to output a variable voltage and/or current (e.g., a sine wave). The processor may be configured to execute the instructions stored on the memory, which may include or define the waveform parameters discussed herein. For example, in some embodiments, the processor may be configured to control the variable power source. The remote control device 70 may control the wearable nerve stimulation device 100 by transmitting pulsed EM energy corresponding to the waveforms 900, 910 shown in Figures 12A and 12B. In other embodiments, the electronic components of the nerve stimulation device may determine one or more characteristics of the waveforms 900, 910.

The devices and systems described herein can be safely used at home and provide invisible therapy options in a background, or on-demand (acute treatment) method. This system may also gather eye position and blink rate data for other data-driven diagnostics. Localized, protected heating through an underlid device does not require invasiveness or anesthetic to be applied as in other prior art systems, and allows for home-based application. Two different secondary hardware devices (frames or handheld external device) allow for two distinct therapy strategies to be applied with the same underlid device: background, continuously applied therapy, or on-demand, manual therapy (acute treatment).

The nerve stimulation devices, systems, and methods described herein may utilize one or more of the components, devices, systems, or methods described in U.S. Patent Application Publication No. 2020/0306537, filed March 25, 2020, and U.S. Patent Application Publication No. 2020/0306538, filed March 25, 2020**.**

Persons skilled in the art will recognize that the devices described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. The invention is defined by the appended claims.

## Claims

1. A device configured to be located underneath an eyelid and worn by a user for treating dry eye, the device comprising:
a flexible substrate;
an antenna disposed on a first side of the substrate and configured to receive electromagnetic energy from a transmitter;
one or more electronic components disposed on the first side of the substrate; and
a first electrode and a second electrode, the first and second electrodes disposed on a second side of the substrate opposite the first side,
wherein the one or more electronic components are in communication with the antenna, the first electrode, and the first electrode, and wherein the one or more electronic components are configured to provide electrical power to the first electrode and the second electrode;
**characterized in that** each of the first electrode and the second electrode comprises an electrode surface and a plurality of slots in the electrode surface defining a plurality of electrode prongs, wherein the plurality of slots facilitate near-field coupling between the transmitter and the antenna.

2. The device of claim 1, wherein the antenna and the one or more electronic components are configured to convert electromagnetic energy into the electrical power.

3. The device of claim 2, wherein the one or more electronic components comprises a rectifier.

4. The device of claim 1, wherein the one or more electronic components comprises an application-specific integrated circuit (ASIC).

5. The device of claim 1, wherein the one or more electronic components comprises one or more discrete surface mount electronic components.

6. The device of claim 1, wherein the first electrode comprises a first plurality of electrode prongs, wherein the second electrode comprises a second plurality of electrode prongs, and wherein the first plurality of electrode prongs and the second plurality of electrode prongs extend in opposing directions.

7. The device of claim 6, wherein the first plurality of electrode prongs and the second plurality of electrode prongs extend toward a central axis of the flexible substrate.

8. The device of claim 6, wherein the first plurality of electrode prongs and the second plurality of electrode prongs extend away from a central axis of the flexible substrate.

9. The device of claim 1, wherein each of the electrode prongs comprises a first width, and wherein each of the slots comprises a second width that is less than the first width.

10. The device of claim 1, wherein the first electrode and the second electrode are spaced from each other on the second side of the substrate.

11. The device of claim 1, further comprising an insulating layer disposed on the first side of the substrate, the insulating layer insulating and sealing the antenna.

12. The device of claim 1, wherein the antenna comprises a first metallic trace arranged in a spiral.

13. The device of claim 12, wherein the antenna defines an antenna region, and wherein the first and second electrodes are juxtaposed on the antenna region.

14. The device of claim 13, wherein the first electrode and the second electrode comprise a second metallic trace, and wherein the second metallic trace is insulated from the first metallic trace by the substrate.

15. A device configured to be located underneath an eyelid and worn by a user for treating dry eye, the device comprising:
a flexible substrate;
an antenna trace disposed on a first side of the substrate and configured to receive electromagnetic energy from a remote control device, the antenna trace comprising at least one loop surrounding and defining an antenna region;
electronic circuitry electrically coupled to the antenna trace; and
a first electrode trace and a second electrode trace electrically coupled to the electronic circuitry, wherein the first and second electrode traces are disposed on a second side of the substrate opposite the first side, and
wherein the first electrode trace and the second electrode trace are juxtaposed with the antenna region,
**characterized in that** each of the first electrode trace and the second electrode trace comprises an electrode surface and a plurality of slots in the electrode surface defining a plurality of electrode branches, wherein the plurality of slots promote near-field coupling between the remote control device and the antenna trace.

16. The device of claim 15, wherein the first electrode trace comprises a first plurality of electrode branches extending in parallel.

17. The device of claim 15, wherein at least a portion of the first electrode trace overlaps with at least a portion of the antenna trace.

18. The device of claim 15, further comprising an elastomeric material encapsulating at least the substrate, the electronic circuitry, and the antenna trace.

19. The device of claim 15, wherein the substrate comprises a bean shape, and wherein device is configured to curl along at least a first axis.

## Patentansprüche

1. Vorrichtung, die ausgelegt ist, um unterhalb eines Augenlids positioniert und durch einen Benutzer zur Behandlung eines trockenen Auges getragen zu werden, wobei die Vorrichtung Folgendes umfasst:
ein flexibles Substrat;
eine Antenne, die auf einer ersten Seite des Substrats angeordnet und ausgelegt ist, um elektromagnetische Energie von einem Sender zu empfangen;
eine oder mehrere elektronische Komponenten, die auf der ersten Seite des Substrats angeordnet sind; und
eine erste Elektrode und eine zweite Elektrode, wobei die erste und die zweite Elektrode auf einer zur ersten Seite entgegengesetzten zweiten Seite des Substrats angeordnet sind,
wobei die eine oder die mehreren elektronischen Komponenten mit der Antenne, der ersten Elektrode und der ersten Elektrode in Kommunikation stehen, und wobei die eine oder die mehreren elektronischen Komponenten ausgelegt sind, um die erste Elektrode und die zweite Elektrode mit elektrischer Leistung zu versorgen;
**dadurch gekennzeichnet, dass** jede aus der ersten Elektrode und der zweiten Elektrode eine Elektrodenoberfläche und eine Vielzahl von Schlitzen in der Elektrodenoberfläche umfassen, die eine Vielzahl von Elektrodenkammzacken definiert, wobei die Vielzahl von Schlitzen eine Nahfeldkopplung zwischen dem Sender und der Antenne unterstützt.

2. Vorrichtung nach Anspruch 1, wobei die Antenne und die eine oder die mehreren elektronischen Komponenten ausgelegt sind, um elektromagnetische Energie in die elektrische Leistung umzuwandeln.

3. Vorrichtung nach Anspruch 2, wobei die eine oder die mehreren elektronischen Komponenten einen Gleichrichter umfassen.

4. Vorrichtung nach Anspruch 1, wobei die eine oder die mehreren elektronischen Komponenten eine anwendungsspezifische integrierte Schaltung (ASIC) umfassen.

5. Vorrichtung nach Anspruch 1, wobei die eine oder die mehreren elektronischen Komponenten eine oder mehrere diskrete oberflächenmontierte elektronische Komponenten umfassen.

6. Vorrichtung nach Anspruch 1, wobei die erste Elektrode eine erste Vielzahl von Elektrodenkammzacken umfasst, wobei die zweite Elektrode eine zweite Vielzahl von Elektrodenkammzacken umfasst und wobei sich die erste Vielzahl von Elektrodenkammzacken und die zweite Vielzahl von Elektrodenkammzacken in entgegengesetzte Richtungen erstrecken.

7. Vorrichtung nach Anspruch 6, wobei sich die erste Vielzahl von Elektrodenkammzacken und die zweite Vielzahl von Elektrodenkammzacken in Richtung einer Mittelachse des flexiblen Substrats erstrecken.

8. Vorrichtung nach Anspruch 6, wobei sich die erste Vielzahl von Elektrodenkammzacken und die zweite Vielzahl von Elektrodenkammzacken von einer Mittelachse des flexiblen Substrats weg erstrecken.

9. Vorrichtung nach Anspruch 1, wobei jeder der Elektrodenkammzacken eine erste Breite umfasst und wobei jeder der Schlitze eine zweite Breite umfasst, die kleiner ist als die erste Breite.

10. Vorrichtung nach Anspruch 1, wobei die erste Elektrode und die zweite Elektrode auf der zweiten Seite des Substrats voneinander beabstandet sind.

11. Vorrichtung nach Anspruch 1, die ferner eine Isolationsschicht umfasst, die auf der ersten Seite des Substrats angeordnet ist, wobei die Isolationsschicht die Antenne isoliert und abdichtet.

12. Vorrichtung nach Anspruch 1, wobei die Antenne eine erste metallische Bahn umfasst, die in einer Spirale angeordnet ist.

13. Vorrichtung nach Anspruch 12, wobei die Antenne einen Antennenbereich definiert und wobei die erste und die zweite Elektrode auf dem Antennenbereich nebeneinander angeordnet sind.

14. Vorrichtung nach Anspruch 13, wobei die erste Elektrode und die zweite Elektrode eine zweite metallische Bahn umfassen und wobei die zweite metallische Bahn durch das Substrat von der ersten metallischen Bahn isoliert ist.

15. Vorrichtung, die ausgelegt ist, um unterhalb eines Augenlids positioniert und durch einen Benutzer zur Behandlung eines trockenen Auges getragen zu werden, wobei die Vorrichtung Folgendes umfasst:
ein flexibles Substrat;
eine Antennenbahn, die auf einer ersten Seite des Substrats angeordnet und ausgelegt ist, um elektromagnetische Energie von einer Fernsteuerungsvorrichtung zu empfangen, wobei die Antennenbahn zumindest eine Schleife umfasst, die einen Antennenbereich umgibt und definiert;
eine elektronische Schaltungsanordnung, die elektrisch mit der Antennenbahn gekoppelt ist; und
eine erste Elektrodenbahn und eine zweite Elektrodenbahn, die elektrisch mit der elektronischen Schaltungsanordnung gekoppelt sind, wobei die erste und die zweite Elektrodenbahn auf einer zweiten Seite des Substrats, entgegengesetzt zur ersten Seite, angeordnet sind, und
wobei die erste Elektrodenbahn und die zweite Elektrodenbahn neben dem Antennenbereich angeordnet sind,
**dadurch gekennzeichnet, dass** jede aus der ersten Elektrodenbahn und der zweiten Elektrodenbahn eine Elektrodenoberfläche und eine Vielzahl von Schlitzen in der Elektrodenoberfläche umfassen, die eine Vielzahl von Elektrodenarmen definiert, wobei die Vielzahl von Schlitzen eine Nahfeldkopplung zwischen der Fernsteuerungsvorrichtung und der Antennenbahn unterstützt.

16. Vorrichtung nach Anspruch 15, wobei die erste Elektrodenbahn eine erste Vielzahl von Elektrodenarmen umfasst, die sich parallel erstrecken.

17. Vorrichtung nach Anspruch 15, wobei zumindest ein Teil der ersten Elektrodenbahn zumindest einen Teil der Antennenbahn überlagert.

18. Vorrichtung nach Anspruch 15, die ferner ein Elastomermaterial umfasst, das zumindest das Substrat, die elektronische Schaltungsanordnung und die Antennenbahn einkapselt.

19. Vorrichtung nach Anspruch 15, wobei das Substrat eine Bohnenform umfasst und wobei die Vorrichtung ausgelegt ist, um sich zumindest entlang einer ersten Achse einzurollen.

## Revendications

1. Dispositif configuré pour être positionné sous une paupière et porté par un utilisateur pour traiter la sécheresse oculaire, le dispositif comprenant :
un substrat flexible ;
une antenne disposée sur un premier côté du substrat et configurée pour recevoir de l'énergie électromagnétique en provenance d'un émetteur ;
un ou plusieurs composants électroniques disposés sur le premier côté du substrat ; et
une première électrode et une seconde électrode, les première et seconde électrodes étant disposées sur un second côté du substrat opposé au premier côté,
dans lequel les un ou plusieurs composants électroniques sont en communication avec l'antenne, la première électrode et la seconde électrode, et dans lequel les un ou plusieurs composants électroniques sont configurés pour fournir de l'énergie électrique à la première électrode et à la seconde électrode ;
**caractérisé en ce que** chacune de la première électrode et de la seconde électrode comprend une surface d'électrode et une pluralité de fentes dans la surface d'électrode définissant une pluralité de broches d'électrode, dans lequel la pluralité de fentes facilite un couplage en champ proche entre l'émetteur et l'antenne.

2. Dispositif selon la revendication 1, dans lequel l'antenne et les un ou plusieurs composants électroniques sont configurés pour convertir de l'énergie électromagnétique en puissance électrique.

3. Dispositif selon la revendication 2, dans lequel les un ou plusieurs composants électroniques comprennent un redresseur.

4. Dispositif selon la revendication 1, dans lequel les un ou plusieurs composants électroniques comprennent un circuit intégré spécifique à une application (ASIC).

5. Dispositif selon la revendication 1, dans lequel les un ou plusieurs composants électroniques comprennent un ou plusieurs composants électroniques à montage en surface discrets.

6. Dispositif selon la revendication 1, dans lequel la première électrode comprend une première pluralité de broches d'électrode, dans lequel la seconde électrode comprend une seconde pluralité de broches d'électrode, et dans lequel la première pluralité de broches d'électrode et la seconde pluralité de broches d'électrode s'étendent dans des directions opposées.

7. Dispositif selon la revendication 6, dans lequel la première pluralité de broches d'électrode et la seconde pluralité de broches d'électrode s'étendent vers un axe central du substrat flexible.

8. Dispositif selon la revendication 6, dans lequel la première pluralité de broches d'électrode et la seconde pluralité de broches d'électrode s'étendent à l'opposé d'un axe central du substrat flexible.

9. Dispositif selon la revendication 1, dans lequel chacune des broches d'électrode comprend une première largeur, et dans lequel chacune des fentes comprend une seconde largeur qui est inférieure à la première largeur.

10. Dispositif selon la revendication 1, dans lequel la première électrode et la seconde électrode sont espacées l'une de l'autre sur le second côté du substrat.

11. Dispositif selon la revendication 1, comprenant en outre une couche isolante disposée sur le premier côté du substrat, la couche isolante isolant et scellant l'antenne.

12. Dispositif selon la revendication 1, dans lequel l'antenne comprend une première trace métallique agencée en une spirale.

13. Dispositif selon la revendication 12, dans lequel l'antenne définit une région d'antenne, et dans lequel les première et seconde électrodes sont juxtaposées sur la région d'antenne.

14. Dispositif selon la revendication 13, dans lequel la première électrode et la seconde électrode comprennent une seconde trace métallique, et dans lequel la seconde trace métallique est isolée de la première trace métallique par le substrat.

15. Dispositif configuré pour être positionné sous une paupière et porté par un utilisateur pour traiter la sécheresse oculaire, le dispositif comprenant :
un substrat flexible ;
une trace d'antenne disposée sur un premier côté du substrat et configurée pour recevoir de l'énergie électromagnétique en provenance d'un dispositif de commande à distance, la trace d'antenne comprenant au moins une boucle entourant et définissant une région d'antenne ;
des circuits électroniques couplés électriquement à la trace d'antenne ; et
une première trace d'électrode et une seconde trace d'électrode couplées électriquement aux circuits électroniques, dans lequel les première et seconde traces d'électrode sont disposées sur un second côté du substrat opposé au premier côté,
dans lequel la première trace d'électrode et la seconde trace d'électrode sont juxtaposées avec la région d'antenne,
**caractérisé en ce que** chacune de la première trace d'électrode et de la seconde trace d'électrode comprend une surface d'électrode et une pluralité de fentes dans la surface d'électrode définissant une pluralité de branches d'électrode, dans lequel la pluralité de fentes favorisent un couplage en champ proche entre le dispositif de commande à distance et la trace d'antenne.

16. Dispositif selon la revendication 15, dans lequel la première trace d'électrode comprend une première pluralité de branches d'électrode s'étendant en parallèle.

17. Dispositif selon la revendication 15, dans lequel au moins une partie de la première trace d'électrode chevauche au moins une partie de la trace d'antenne.

18. Dispositif selon la revendication 15, comprenant en outre un matériau élastomère encapsulant au moins le substrat, les circuits électroniques et la trace d'antenne.

19. Dispositif selon la revendication 15, dans lequel le substrat présente une forme de haricot, et dans lequel le dispositif est configuré pour se courber le long d'au moins un premier axe.
